# EUROPÄISCHE PATENTSCHRIFT

(11) **EP 0 470 103 B1**
(45) Veröffentlichungstag und Bekanntmachung des Hinweises auf die Patenterteilung: **27.07.1994**
(21) Anmeldenummer: 90906184.8
(22) Anmeldetag: 21.04.1990
(51) Int. Cl.: C07C 317/44, C07C 315/04

(54) **VERFAHREN ZUR HERSTELLUNG VON ALKANSULFONYLBENZOESÄUREN**
PROCESS FOR THE PRODUCTION OF ALKANE SULPHONYL BENZOIC ACIDS
PROCEDE DE FABRICATION D'ACIDES ALCANE-SULFONYLBENZOIQUES

(30) Priorität: 29.04.1989 DE 3914390
(43) Veröffentlichungstag der Anmeldung: 12.02.1992
(73) Patentinhaber: HOECHST AKTIENGESELLSCHAFT, 65926 Frankfurt am Main (DE)
(72) Erfinder: RÖHRSCHEID, Freimund, D-6233 Kelkheim/Taunus (DE)
(86) Internationale Anmeldenummer: EP9000646
(87) Internationale Veröffentlichungsnummer: WO9013537

(56) Entgegenhaltungen:
- US-A- 3 692 828

## Beschreibung

Alkansulfonylbenzoesäuren sind Vorprodukte für Schädlingsbekämpfungsmittel und Herbizide (US-PS 4.704.467, EP-OS 203 428). Sie werden nach dem bisherigen Stand der Technik z.B. durch Oxydation von Alkylthiobenzoesäuren mit einem Oxydationsmittel wie Natriumhypochlorit oder H₂O₂ hergestellt. In der US-PS 4.704.467 wird für die Methansulfonylbenzoesäure folgender Weg beschrieben: 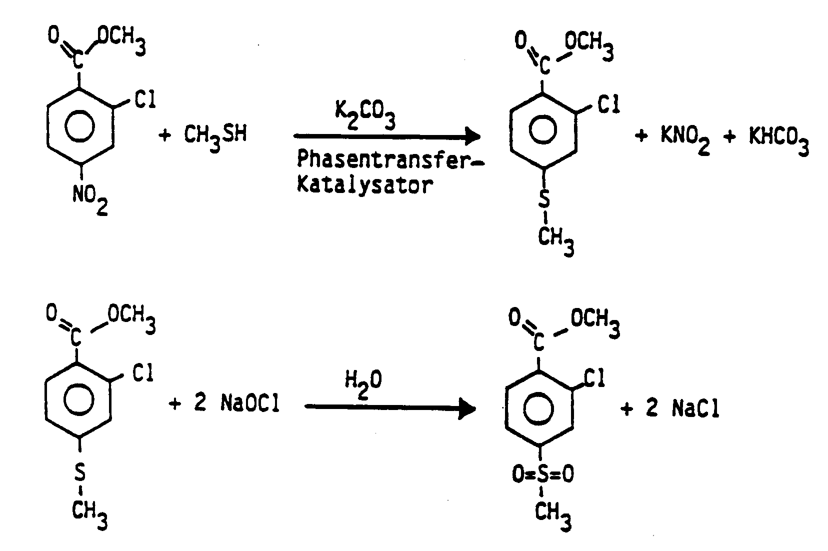
Nachteile dieser Verfahrensweise sind die umständliche Einführung des Alkylthiorestes, das Arbeiten mit den geruchsintensiven Mercaptanen und die Oxydation mit Chemikalien wie Hypochlorit oder H₂O₂.

Es wurde nun überraschend gefunden, daß sich Alkansulfonylbenzoesäuren in einfacher Weise herstellen lassen, wenn man Alkansulfonyl-alkyl-benzole der allgemeinen Formel I (s. Patentanspruch 1), worin R¹ und R² gleich oder verschieden und Alkyl mit 1 bis 4 C-Atomen sind, R² jedoch eine andere Bedeutung als t-Butyl hat, und X für H, F, Cl, Br oder NO₂ steht, mit molekularem Sauerstoff in Essig- und/oder Propionsäure in Gegenwart eines darin löslichen Katalysators, der Kobalt- und Bromionen und, wenn R² eine andere Bedeutung als Methyl hat, auch Manganionen enthält, unter erhöhtem Druck bei Temperaturen oberhalb 120°C oxydiert.

Als Alkylgruppen für R¹ und R² kommen jeweils Methyl-, Äthyl, n- und iso-Propyl, n-, sek- und iso-Butyl in Frage, für R¹ zusätzlich auch t-Butyl. Bei der Oxydation wird der Rest R² zur COOH-Gruppe oxydiert. Zur Oxydation der Methylgruppe R² sind 1,5 Mol Sauerstoff erforderlich.

Die Ausgangsverbindungen sind leicht zugänglich; z.B. lassen sich Alkansulfonyltoluole aus Toluolsulfonsäure durch Reduktion zum toluolsulfinsauren Natrium und dessen Umsetzung mit Dimethylsulfat oder Diäthylsulfat herstellen.

Geeignete Ausgangsverbindungen sind außer den in den Beispielen genannten Verbindungen z. B. 2-[Methansulfonyl]-5-nitrotoluol, 3-Nitro-4-[methansulfonyl]-toluol und 6-Chlor-3-nitro-4-[methansulfonyl]-toluol.

Das vorliegende Verfahren wird vorteilhaft unter den für die Oxydation von Alkylgruppen besonders wirksamen Bedingungen, wie sie z.B. für die Oxydation beider Methylgruppen des p-Xylols zur Terephthalsäure angewendet werden, durchgeführt. Durch Anwendung dieser sehr wirksamen Oxydationsbedingungen ist es möglich, die Alkylgruppe am Benzolring zu oxydieren, auch wenn der Benzolring zusätzlich zur stark elektronenziehenden Alkansulfonylgruppe einen zusätzlichen desaktivierenden Substituenten X trägt. Es ist jedoch überraschend, daß unter diesen Oxydationsbedingungen nur die Alkylgruppe am Benzolring oxydiert wird, die Alkansulfonylgruppe dagegen erhalten bleibt. So wird im Gegensatz zur Alkansulfonylgruppe z.B. die Acetylgruppe des Acetophenons unter diesen Bedingungen leicht oxydativ abgebaut.

Die Essig- und/oder Propionsäure wird im allgemeinen in wasserfreier Form eingesetzt; jedoch bildet sich bei der Oxydation Wasser. Das Reaktionssystem enthält jedoch im allgemeinen nicht mehr als 15 Gew.-% Wasser, insbesondere höchstens 5 Gew.-%. Die Verwendung von Essigsäure, insbesondere in wasserfreier Form, ist bevorzugt.

Die Anwesenheit von Manganionen ist für die gewünschte Oxydation der am Aromaten gebundenen Äthyl-, Propyl- und Butylgruppe notwendig, aber auch für die Oxydation der Methylgruppe zweckmäßig, wenngleich für diese letztere auch eine Kombination aus Kobalt- und Bromionen genügt. Die Gegenwart von Manganionen ermöglicht eine Verringerung der erforderlichen Kobaltmenge bei gleicher Aktivität des Katalysators.

Das Verhältnis der Konzentration von Kobalt- zu Manganionen beträgt im allgemeinen 1:(0,2 bis 3), bevorzugt 1:(0,3 bis 1,2). Das Verhältnis der Summe der Konzentrationen beider Metallionen zu Bromionen beträgt zweckmäßig 1:(0,01 bis 2), vorzugsweise 1:(0,1 bis 1), besonders bevorzugt 1:(0,2 bis 0,7). Die Summe der Konzentrationen beider Metallionen liegt zweckmäßig im Bereich von 0,01 bis 0,2 Mol, vorzugsweise von 0,02 bis 0,1 Mol und insbesondere von 0,04 bis 0,08 Mol Metallionen je 1 der flüssigen Phase. Die Metallionen werden bevorzugt in Form der Salze der betreffenden Carbonsäure zugefügt. Das Bromid kann in Form einer HBr-Lösung, als Alkalibromid oder zweckmäßig als Kobalt- oder Manganbromid zugegeben werden.

Der molekulare Sauerstoff wird bevorzugt in Form trockener Luft in die flüssige Phase des Reaktors eingeführt. Das Verfahren wird zweckmäßig unter einem Sauerstoffpartialdruck, von 1,5 bis 8, vorzugsweise von 2,4 bis 7 und insbesondere von 2,8 bis 6 bar durchgeführt. Die Reaktionstemperatur liegt zweckmäßig im Bereich von 120 bis 220°C, vorzugsweise von 130 bis 180°C, und insbesondere von 135 bis 160°C.

Für die vollständige und rasche Oxydation der Alkylgruppe am Benzolring ist es ganz besonders vorteilhaft, bei einer Katalysatorkonzentration von 0,04 bis 0,08 Mol Metallionen pro 1 flüssiger Phase zu arbeiten und an der Eintrittsstelle in die flüssige Phase einen hohen Sauerstoffpartialdruck von mindestens 2,4 bzw. 2,5 bar anzuwenden. Die Kombination aus diesen beiden Maßnahmen ermöglicht es auch, die erforderliche Reaktionstemperatur niedrig zu halten. Wenn man keine extrem scharfen Bedingungen kombiniert, wird die Alkansulfonylgruppe nur unerheblich oxydativ angegriffen.

Die Alkansulfonylsäuren kristallisieren je nach Konzentration beim Abkühlen aus der Reaktionslösung aus und können durch Filtration abgetrennt werden. Im Filtrat befinden sich aber oft erhebliche Mengen an gelöstem Produkt und der gesamte Katalysator. Eine vorteilhafte Ausführungsform des Verfahrens besteht deshalb darin, aus dem Filtrat, z. B. an einer Kolonne, während der Reaktion entstandenes Wasser abzutrennen und diese Mutterlauge wieder als Reaktionsmedium für die nächste Oxydation zu verwenden. Dadurch wird Katalysator eingespart, und die isolierte Ausbeute des darauffolgenden Ansatzes ist höher.

In den folgenden Beispielen steht "OAc" für Acetat.

### Beispiele

1) In einem 1 l-Autoklaven aus Edelstahl, der mit Thermometer, Rührer, Rückflußkühler und Druckhalteventil ausgestattet war, wurde eine Mischung aus 245,6 g 2-Chlor-4-methansulfonyltoluol, 12,0 g Co(OAc)₂ · 4H₂O, 16,2 g einer 10%igen Bromwasserstoff-Lösung in Eisessig und 440 g Eisessig unter 16 bar Stickstoff auf 150°C erhitzt. Dann wurde Luft (16 bar) über ein Tauchrohr in die flüssige Phase eingeleitet. Die exotherme Reaktion setzte sofort ein, und durch Kühlung wurde die Temperatur bei 155 bis 160°C gehalten. Durch Regelung der Luftzufuhr wurde der Sauerstoffgehalt im Abgas bei 5 bis 6 Vol-% gehalten. Nach Ende der exothermen Reaktion wurde die heiße Reaktionslösung entnommen und unter Rühren auf 20°C abgekühlt. Die ausgefallenen Kristalle wurden abgesaugt, fünf Mal mit je 30 ml Eisessig gewaschen und bei 80°C und 65 mbar im schwachem Luftstrom getrocknet.
   - Ausbeute:: 243,6 g (86,5 % d.Th.) 2-Chlor-4-(methansulfonyl)-benzoesäure; Fp 193 bis 194°C
2) In dem im Beispiel 1 beschriebenen Autoklaven wurde eine Mischung aus 221,3 g 3-(Methansulfonyl)-toluol, 7,5 g Co(OAc)₂ · 4H₂O, 2,45 g Mn(OAc)₂ · 4H₂O, 16,2 g einer 10%igen Bromwasserstoff-Lösung in Eisessig und 470 g Eisessig bei 150 bis 155°C und 15 bar mit Luft oxydiert. Nach Ende der Sauerstoffaufnahme wurden der 100°C heißen Reaktionslösung zwecks Ausfällung des Reaktionsproduktes 200 g Wasser zugefügt und die Mischung auf 20°C abgekühlt. Die Kristalle wurden abgesaugt, fünf Mal mit je 30 ml 70%iger wäßriger Essigsäure gewaschen und bei 80°C und 65 mbar in schwachem Luftstrom getrocknet.
   - Ausbeute:: 236,7 g (91 % d.Th.) 3-(Methansulfonyl)-benzoesäure; Fp 236 bis 237°C.
3) In dem im Beispiel 1 beschriebenen Autoklaven wurde eine Mischung aus 221,1 g 4-(Methansulfonyl)-1-äthylbenzol, 5,0 g Co(OAc)₂ · 4H₂O, 4,9 g Mn(OAc)₂ · 4H₂O, 12,2 g einer 10%igen Bromwasserstoff-Lösung in Eisessig und 480 g Eisessig bei 145 bis 150°C und 16 bar mit Luft oxydiert. Nach Ende der Sauerstoffaufnahme wurde unter Rühren auf 20°C gekühlt. Die Kristalle wurde scharf abgesaugt, fünf Mal mit je 30 ml Eisessig gewaschen und bei 100°C und 65 mbar im leichten Luftstrom getrocknet.
   - Ausbeute:: 223,9 g (93,2 % d.Th.) 4-Methansulfonyl-benzoesäure; Fp 273 bis 274°C.
4) In dem im Beispiel 1 beschriebenen Autoklaven wurde eine Mischung aus 198,3 g (1,0 Mol) 1-Propyl-4-tolylsulfon, 7,5 g Co(OAc)₂ · 4H₂O, 2,45 g Mn(OAc)₂ · 4H₂O, 8,1 g einer 10%igen Bromwasserstoff-Lösung in Eisessig und 500 g Eisessig bei 145 bis 150°C und 20 bar mit Luft oxydiert. Nach Ende der Sauerstoffaufnahme wurden bei 100°C 200 g Wasser zugefügt und das Gemisch unter Rühren auf 20°C abgekühlt. Die Kristalle wurden scharf abgesaugt, fünf Mal mit je 30 ml 70%iger wäßriger Essigsäure gewaschen und bei 80°C und 65 mbar im leichten Luftstrom getrocknet.
   - Ausbeute:: 189,0 g (82,8 % d.Th.) 4-(Propan-1-sulfonyl)-benzoesäure; Fp 196 bis 198°C.

## Patentansprüche

1. Verfahren zur Herstellung von Alkansulfonyl-benzoesäuren, dadurch gekennzeichnet, daß man Alkansulfonyl-alkyl-benzole der Formel 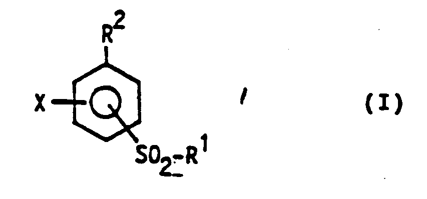 worin R¹ und R² gleich oder verschieden und Alkyl mit 1 bis 4 C-Atomen sind, R² jedoch eine andere Bedeutung als t-Butyl hat, und X für H, F, Cl, Br oder NO₂ steht, mit molekularem Sauerstoff in Essig- und/oder Propionsäure in Gegenwart eines darin löslichen Katalysators, der Kobalt- und Bromionen und, insbesondere wenn R² eine andere Bedeutung als Methyl hat, auch Manganionen enthält, unter erhöhtem Druck bei Temperaturen oberhalb 120°C oxydiert.

2. Verfahren nach Anspruch 1, dadurch gekennzeichnet, daß das Reaktionssystem nicht mehr als 15, insbesondere nicht mehr als 5 Gew.% Wasser enthält.

3. Verfahren nach Anspruch 1 oder 2, dadurch gekennzeichnet, daß Essigsäure verwendet wird, vorzugsweise in wasserfreier Form.

4. Verfahren nach einem oder mehreren der Ansprüche 1 bis 3, dadurch gekennzeichnet, daß Kobalt- und Mangan-ionen im Konzentrationsverhältnis 1:(0,2 bis 3), vorzugsweise 1:(0,3 bis 1,2), verwendet werden.

5. Verfahren nach einem oder mehreren der Ansprüche 1 bis 4, dadurch gekennzeichnet, daß das Verhältnis der Summe der Konzentrationen von Kobalt- und Mangan-ionen und der Konzentration der Bromionen 1:(0,01 bis 2), vorzugsweise 1:(0,1 bis 1) und insbesondere 1:(0,2 bis 0,7) beträgt.

6. Verfahren nach einem oder mehreren der Ansprüche 1 bis 5, dadurch gekennzeichnet, daß die Summe der Konzentrationen beider Metallionen 0,01 bis 0,2 Mol, vorzugsweise 0,02 bis 0,1 Mol, besonders bevorzugt 0,04 bis 0,08 Mol Metallionen je l flüssiger Phase beträgt.

7. Verfahren nach einem oder mehreren der Ansprüche 1 bis 6, dadurch gekennzeichnet, daß der Sauerstoffpartialdruck 1,5 bis 8, vorzugsweise 2,4 bis 7 und insbesondere 2,8 bis 6 bar beträgt.

8. Verfahren nach einem oder mehreren der Ansprüche 1 bis 7, dadurch gekennzeichnet, daß die Reaktionstemperatur 120 bis 220°C, vorzugsweise 130 bis 180°C und insbesondere 135 bis 160°C beträgt.

9. Verfahren nach einem oder mehreren der Ansprüche 1 bis 8, dadurch gekennzeichnet, daß ausgefallenes kristallines Produkt abgetrennt, während der Reaktion entstandenes Wasser entfernt und so erhaltene Mutterlauge erneut als Reaktionsmedium eingesetzt wird.

## Claims

1. Process for the preparation of alkanesulfonyl-benzoic acids, characterized in that alkanesulfonyl-alkyl-benzenes of the formula 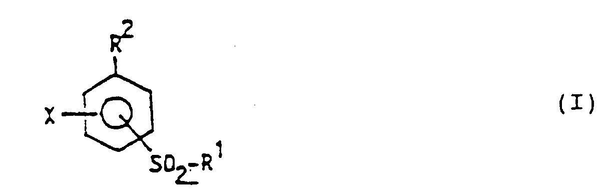 in which R¹ and R² are identical or different and are alkyl having 1 to 4 carbon atoms, but R² has a meaning other than t-butyl, and X represents H, F, Cl, Br or NO₂, are oxidized under increased pressure at temperatures above 120°C with molecular oxygen in acetic and/or propionic acid in the presence of a catalyst soluble therein, which contains cobalt ions and bromine ions and, in particular if R² has a meaning other than methyl, also manganese ions.

2. Process according to Claim 1, characterized in that the reaction system contains not more than 15, in particular not more than 5 % by weight of water.

3. Process according to Claim 1 or 2, characterized in that acetic acid is used, preferably in anhydrous form.

4. Process according to one or more of Claims 1 to 3, characterized in that cobalt ions and manganese ions are used in a concentration ratio of 1:(0.2 to 3), preferably 1:(0.3 to 1.2).

5. Process according to one or more of Claims 1 to 4, characterized in that the ratio of the sum of the concentrations of cobalt ions and manganese ions and the concentration of bromine ions is 1:(0.01 to 2), preferably 1:(0.1 to 1) and in particular 1:(0.2 to 0.7).

6. Process according to one or more of Claims 1 to 5, characterized in that the sum of the concentrations of the two metal ions is 0.01 to 0.2 mol, preferably 0.02 to 0.1 mol and particularly preferably 0.04 to 0.08 mol of metal ions per l of liquid phase.

7. Process according to one or more of Claims 1 to 6, characterized in that the oxygen partial pressure is 1.5 to 8, preferably 2.4 to 7 and in particular 2.8 to 6 bar.

8. Process according to one or more of Claims 1 to 7, characterized in that the reaction temperature is 120 to 220°C, preferably 130 to 180°C and in particular 135 to 160°C.

9. Process according to one or more of Claims 1 to 8, characterized in that the crystalline product which has precipitated is separated off, the water formed during the reaction is removed and the mother liquor thus obtained is used again as the reaction medium.

## Revendications

1. Procédé de préparation d' acides alcanesulfonylbenzoïques, caractérisé en ce que l'on oxyde un alcanesulfonylalkylbenzène de formule : 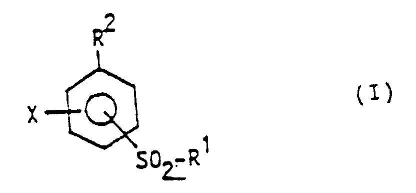 dans laquelle R¹ et R² sont identiques ou différents et représentent des groupes alkyles comportant de 1 à 4 atomes de carbone, R² étant cependant différent du groupe tertbutyle, dans laquelle X peut être soit un atome d'hydrogène, soit le fluor (F), le chlore (Cl) le brome (Br), soit un groupe NO₂, par l'oxygène moléculaire dans l'acide acétique et/ou l'acide propionique et en présence d'un catalyseur soluble dans le mélange et qui comprend des ions de cobalt et de brome et, lorsque R² est différent du groupe méthyle, comprend en outre des ions de manganèse, sous une pression élevée et à une température supérieure à 120°C.

2. Procédé selon la revendication 1, caractérisé en ce que le système réactionnel ne contient pas plus de 15 % en poids d'eau et, plus particulièrement pas plus de 5 %.

3. Procédé selon l'une quelconque des revendications 1 ou 2, caractérisé en ce que l'on utilise, de préférence, l'acide acétique sous une forme anhydre.

4. Procédé selon l'une ou plusieurs des revendications 1 à 3, caractérisé en ce que l'on utilise les ions de cobalt et de manganèse dans un rapport de concentration de 1:(0,2 à 3), de préférence de 1:(0,3 à 1,2).

5. Procédé selon l'une ou plusieurs des revendications 1 à 4, caractérisé en ce que le rapport de la somme des concentrations des ions de cobalt et de manganèse à la concentration des ions de brome s'élève à 1:(0,01 à 2), de préférence à 1:(0,1 à 1), d'une manière encore préférée à 1:(0,2 à 0,7).

6. Procédé selon l'une ou plusieurs des revendications 1 à 5, caractérisé en ce que la somme des concentrations des deux ions métalliques est comprise entre 0,01 et 0,2 mole, de préférence entre 0,02 et 0,1 mole, d'une manière encore préférée entre 0,04 et 0,08 mole d'ions métalliques pour un litre de phase liquide.

7. Procédé selon l'une ou plusieurs des revendications 1 à 6, caractérisé en ce que la pression partielle d'oxygène est comprise entre 1,5 et 8, de préférence entre 2,4 et 7, d'une manière encore préférée entre 2,8 et 6 bars.

8. Procédé selon l'une ou plusieurs des revendications 1 à 7, caractérisé en ce que la température de la réaction est comprise entre 120 et 220°C, de préférence entre 130 et 180°C, d'une manière encore préférée entre 135 et 160°C.

9. Procédé selon l'une ou plusieurs des revendications 1 à 8, caractérisé en ce que l'on effectue les opérations qui consistent à séparer le produit cristallin précipité, éliminer l'eau produite pendant la réaction, et utiliser une nouvelle fois la solution mère ainsi obtenue comme milieu réactionnel.
